(19) Europäisches Patentamt
European Patent Office
Office européen des brevets

(11) Veröffentlichungsnummer: **0 003 029**
**B1**

(12) # EUROPÄISCHE PATENTSCHRIFT

(21) Anmeldenummer: **78101627.4**

(22) Anmeldetag: **09.12.78**

(51) Int. Cl.[3]: **C 07 D 207/26,**
C 07 D 403/06,
C 07 D 401/06,
A 61 K 31/40

(54) **Neue 2-Oxo-1-pyrrolidinessigsäurederivate, Verfahren zu ihrer Herstellung, diese enthaltende Arzneimittel und deren Herstellung**

(30) Priorität: **23.12.77 DE 2757680**

(43) Veröffentlichungstag der Anmeldung:
**25.07.79 Patentblatt 79/15**

(45) Bekanntmachung des Hinweises auf die Patenterteilung:
**10.12.80 Patentblatt 80/25**

(84) Benannte Vertragsstaaten:
**BE CH DE FR GB IT NL**

(56) Entgegenhaltungen:
**US - A - 3 766 302**

(73) Patentinhaber: **Troponwerke GmbH & Co. KG**
**Berliner Strasse 156**
**D - 5000 Köln 80 (DE)**

(72) Erfinder: **Boltze, Karl-Heinz, Dr.**
**Am Burgtor 23**
**D - 5060 Bergisch Gladbach 3 (DE)**
**Dell, Hans-Dieter, Dr.**
**Kalmüntener Strasse 5**
**D - 5060 Bergisch Gladbach 2 (DE)**
**Jacobi, Haireddin, Dr.**
**Finkenweg 2**
**D - 5652 Leichlingen (DE)**

(74) Vertreter: **Dill, Erwin, Dr., et al**
**c/o BAYER AG Zentralbereich Patente Marken und Lizenzen Bayerwerk**
**D - 5090 Leverkusen 1 (DE)**

**EP 0 003 029 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Neue 2-Oxo-1-pyrrolidinessigsäurederivate, Verfahren zu ihrer Herstllung, diese enthaltende Arzneimittel und deren Herstellung

Die Erfindung betrifft neue 2-Oxo-1-pyrrolidinessigsäurederivate, ein Verfahren zu ihrer Herstellung, Arzneimittel enthaltend diese Verbindungen und Verfahren zur Herstellung dieser Arzneimittel.

Die Erfindung betrifft neue 2-Oxo-1-pyrrolidinessigsäurederivate der allgemeinen Formel

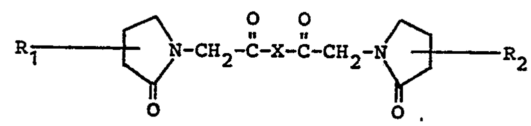

(I)

in welcher

$R_1$ und $R_2$ jeweils für Wasserstoff, Methyl, Methoxy, Fluor oder Chlor stehen,

X für die Gruppierung $-\underset{\displaystyle R_3}{\underset{|}{N}}-Y-\underset{\displaystyle R_4}{\underset{|}{N}}-$ steht;

wobei diese Gruppierung einschließlich der beiden Stickstoffatome entweder für einen Heterozyklus mit 5,6 oder 7 Ringgliedern steht,
oder wobei

$R_3$ und $R_4$ jeweils Wasserstoff oder Methyl bedeutet und

Y für eine Phenylen- oder eine Pyridylengruppe steht
oder für eine Alkylenarylenalkylengruppierung steht, in welcher die Alkylengruppen 1 oder 2 Kohlenstoffatome enthalten und die Arylengruppe eine Phenylen- oder Pyridylengruppe bedeutet
oder für eine Cycloalkylengruppe mit 5,6 oder 7 Ringgliedern steht
oder für eine geradkettige oder verzweigte Alkylengruppe mit 2 bis 9 Kohlenstoffatomen steht die gegebenenfalls durch eine Äthoxycarbonylgruppe substituiert ist und in welcher gegebenenfalls eine $CH_2$-Gruppe durch eine N-Alkyl (1—2 C-Atome) substituiert ist,

und ihre physiologisch verträglichen Salze mit Säuren.

Es wurde gefunden, daß man 2-Oxo-1-pyrrolidinessigsäurederivate der allgemeinen Formel (I), in welcher $R_1$ bis $R_4$ X und Y die angeführte Bedeutung haben, erhält, wenn man Verbindungen der allgemeinen Formel

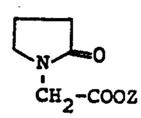

(II)

in welcher

Z eine niedere Alkylgruppe oder eine aktivierte Estergruppierung, bedeutet,

mit einer Verbindung der allgemeinen Formel

$$H—X—H \qquad (III)$$

in welcher

X die oben angeführte Bedeutung hat,

gegebenenfalls in Gegenwart von inerten organischen Lösungsmitteln und gegebenenfalls in Gegenwart einer Base, umsetzt.

Überraschenderweise zeigen die erfindungsgemäßen 2-Oxo-1-pyrrolidinessigsäurederivate eine bessere Wirksamkeit als Psychopharmaka als vergleichbare Wirkstoffe aus dem Stand der Technik, z.B. das bekannte 2-Oxo-1-pyrrolidinacetamid (I.N.N.: Piracetam). Die erfindungsgemäßen Stoffe stellen somit eine Bereicherung der Pharmazie dar, insbesondere aufgrund ihrer guten nootropen Wirkung.

Verwendet man für die Reaktion Verbindungen der allgemeinen Formel (II), in welcher Z für eine niedere Alkylgruppe steht, wie z.B. den Äthylester, so kann der Reaktionsablauf durch das folgende Formelschema wiedergegeben werden:

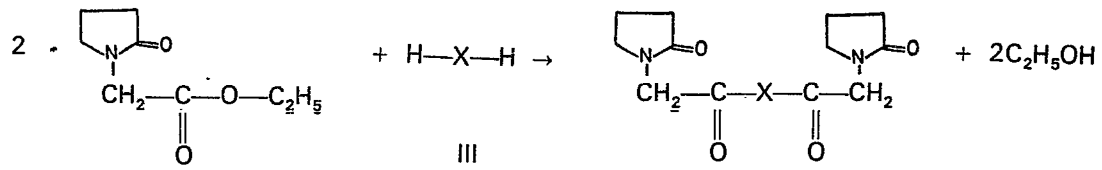


III

I

II

Die Reaktion wird zweckmäßig in Gegenwart von Verdünnungsmitteln durchgeführt. Als Verdünnungsmittel kommen alle inerten organischen Lösungsmittel in Frage, in denen sich die Reaktionspartner lösen. Beispielhaft seien genannt Alkohole wie Methanol, Äthanol, Propanol oder Isopropanol, Äther wie Diäthyläther, Diglym oder Dioxan oder Dimethylformamid.

Die Reaktionstemperaturen können in einem größeren Bereich variiert werden. Im allgemeinen arbeitet man bei der Siedetemperatur des jeweiligen Lösungsmittels.

Temperatur und Lösungsmittel hängen außerdem von der Reaktionsfähigkeit des jeweiligen Amins der allgemeinen Formel (III) ab. Bei sehr trägen Aminen hat es sich als zweckmäßig erwiesen, anstelle des Äthylesters der allgemeinen Formel (II) einen aktivierten Ester, beispielhaft den Trichlorphenylester zu verwenden.

Auch hier wird zweckmäßig mit Verdünnungsmitteln gearbeitet. Als Verdünnungsmittel kommen alle organischen Lösungsmittel in Frage in welchen sich die Reaktionspartner wenigstens teilweise lösen. Beispielhaft seien genannt Dichlormethan, Chloroform, Dioxan, Diglym, Morpholin, Pyridin, Dimethylformamid, Äthylacetat, Methylacetat; als besonders geeignet hat sich Dimethylformamid erwiesen.

Die Reaktionstemperaturen liegen zwischen $-20°C$ und $+60°C$, vorzugsweise zwischen $-5°C$ und $+30°C$. Im allgemeinen arbeitet man bei Normaldruck.

Bei der Durchführung des erfindungsgemäßen Verfahrens setzt man pro Mol der jeweiligen Verbindung der allgemeinen Formel (II) 0,5 Mol des jeweiligen Amins der allgemeinen Formel (III) ein.

Die Reaktionszeit ist unterschiedlich und liegt je nach Reaktionstemperatur zwischen fünf und zwanzig Stunden.

Die Aufbereitung erfolgt im allgemeinen durch Abdampfen des Lösungsmittels und Chromatographie aus einen geeigneten Laufmittelgemisch an Kieselgel.

Der 2,4,5-Trichlorphenylester von 2-Oxo-1-pyrrolidinessigsäure ist bisher nicht in der Literatur beschrieben worden. Er entsteht in an sich bekannter Weise durch Reaktion des 2,4,5-Trichlorphenols mit der vorstehend genannten Säure in einem geeigneten Lösungsmittel in Gegenwart von Dicyclohexylcarbodiimid.

Als neue Wirkstoffe seien im einzelnen genannt:
1,2-Bis(2-oxo-1-pyrrolidinacetamido)äthan,
1,6-Bis(2-oxo-1-pyrrolidinacetamido)hexan,
1,4-Bis(2-oxo-1-pyrrolidinacetyl)piperazin,
N-Methyl-N,N′-bis(2-oxo-1-pyrrolidinacetyl)äthylendiamin,
N,N′-Dimethyl-N,N′-bis-(2-oxo-1-pyrrolidinacetyl)äthylendiamin,
4-Methyl-1,7-bis(2-oxo-1-pyrrolidinacetyl)-4-aza-1,7-diaminoheptan,
2,2,4-Trimethyl-1,6-bis(2-oxo-1-pyrrolidinacetyl)-1,6-diaminohexan,
1,4-Bis(2-oxo-1-pyrrolidinacetamido)cyclohexan,
$\alpha,\alpha'$-Bis(2-oxo-1-pyrrolidinacetamido)-m-xylol,
1,2-Bis(2-oxo-1-pyrrolidinacetyl)-1,2-propylendiamin.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Supposotorien, Lösungen, Suspensionen und Emulsionen genannt.

Tabletten, Dragees, Kapseln, Pillen und Granulate können den oder die Wirkstoffe neben dem üblichen Trägerstoffen enthalten, wie Füll- und Streckmittel (z.B. Stärken, Milchzucker, Rohrzucker,

Glukose, Mannit und Kieselsäure), Bindemittel (z.B. Carboxymethylcellulose, Alginate, Gelatine, Polyvinylpyrrolidon), Feuchthaltemittel (z.B. Glycerin), Sprengmittel (z.B. Agar-Agar, Calciumcarbonat und Natriumbicarbonat). Resorptionsbeschleuniger (z.B. quaternäre Ammoniumverbindungen), Netzmittel (z.B. Cetylalkohol, Glycerinmonostearat), Adsorptionsmittel (z.B. Kaolin und Bentonit), Gleitmittel (z.B. Talkum, Calcium- und Magnesiumstearat) und feste Polyäthylenglykole oder Gemische der aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes abgeben, wobei als Einbettungsmassen z.B. Polymersubstanzen und Wachse verwendet werden können.

Der oder die Wirkstoffe können mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Proplenglykol, 1,3-Butylenglykol, Dimethylformamid, Öle, insbesondere Baumwollsaatöl, Erdnußöl, Maiskeimöl, Olivenöl Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummethahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, insbesondere von etwa 0,5 bis 95 Gew.-% der Gesamtmischung vorhanden sein.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können oral, parenteral, intraperitoneal und/oder rectal, vorzugsweise oral appliziert werden.

Im allgemeinen hat es sich in der Humanmedizin als vorteilhaft erwiesen, den oder die erfindungsgemäßen Wirkstoffe in Gesamtmengen von etwa 1 bis etwa 100, vorzugsweise 10 bis 50 mg/kg Körpergewicht je 24 Stunden, gegebenenfalls in Form mehrer Einzelgaben, zur Erzielung der gewünschten Ergebnisse zu verabreichen. Eine Einzelgabe enthält den oder die erfindungsgemäßen Wirkstoffe vorzugsweise in Mengen von etwa 0,5 bis 50, insbesondere 3 bis 30 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens erfolgen.

## Beispiel 1

### 1,2-Bis-(2-oxo-1-pyrrolidinacetamido)äthan

17,1 g (0,1 Mol) Äthyl-2-oxo-1-pyrrolidinacetat und 3 g (0,05 Mol) 1,2-Äthylendiamin werden in 30 ml Isopropanol gelöst und unter Feuchtigkeitsausschluß 8 Stunden gekocht. Der beim Abkühlen ausfallende Niederschlag (6,5 g) wird aus dem Laufmittelgemisch Methanol/$NH_4OH$ (9:1) an Kieselgel chromatographiert.

Fp.: 212—213°C, Ausbeute: 5,75 g (35% der Theorie)

$C_{14}H_{22}N_4O_4$ (310,35)

| | | | |
|---|---|---|---|
| berechnet: | C 54,18%, | H 7,14%, | N 18,05%; |
| gefunden: | C 54,11%, | H 7,01%, | N 17,98%. |

Daneben aus dem vorstehend beschriebenen Chromatogramm als ölige Verbindung 2,9 g
*2-Aminoäthyl-(2-oxo-1-pyrrolin) acetamid*
p-Aminobenzoat, Fp. 149—150°C.

## Beispiel 2

1,6-Bis(2-oxo-1-pyrrolidinacetamido)hexan

10 g (0,06 Mol) Äthyl-2-oxo-1-pyrrolidinacetat und 3,4 g (0,03 Mol) 1,6-Diaminohexan werden in 5 ml absolutem Methanol gelöst und 19 Stunden lang unter Feuchtigkeitsausschlußgekocht. Die Lösung wird mit Äther versetzt und der ausfallende Niederschlag abfiltriert. Die Reinigung erfolgt durch Chromatographie aus Methanol:Wasser (100:25) an Kieselgel.

Fp.: 122°C, Ausbeute: 8,2 g (74% der Theorie).
$C_{18}H_{30}N_4O_4$ (366,47)
berechnet: C 58,99%, H 8,25%, N 15,29%;
gefunden: C 58,98%, H 8,36%, N 15,18%.

## Beispiel 3

1,4-Bis(2-oxo-1-pyrrolidinacetyl)piperazin

Aus 10 g (0,06 Mol) Äthyl-2-oxo-1-pyrrolidinacetat und 2,5 g (0,03 Mol) Piperazin, analog Beispiel 2.

Fp.: 292°C, Ausbeute: 1,1 g (11% der Theorie).
$C_{16}H_{24}N_4O_4$ (336,40)
berechnet: C 57,12%, H 7,19%, N 16,65%;
gefunden: C 57,13%, H 7,31%, N 16,40%.

## Beispiel 4

N-Methyl-N,N'-bis(2-oxo-1-pyrrolidinacetyl)äthylendiamin

Analog Beispiel 2 aus 8,55 g (0,05 Mol) Äthyl-2-oxo-1-pyrrolidinacetat und 1,85 g (0,025 Mol) N-Methylaminoäthylamin. Es handelt wich um ein öliges Produkt von $n_D^{54}$ 1,5205; Ausbeute: 1 g (40% der Theorie).

$C_{15}H_{24}N_4O_4$ (324,39)
berechnet: C 55,54%, H 7,45%, N 17,27%;
gefunden: C 55,23%, H 7,75%, N 17,28%.

## Beispiel 5

N,N'-Dimethyl-N,N'-bis(oxo-1-pyrrolidinacetyl)-äthylendiamin

12,88 g (0,04 Mol) 2,3,5-Trichlorphenyl-2-oxo-1-pyrrolidinacetat werden in 200 ml absolutem Dimethylformamid gelöst und 15 Stunden lang, zuletzt bei Raumtemperatur, aufbewahrt. Nach dem Abdampfen des Lösungsmittels wird der Rückstand mit Petroläther ausgewaschen, der unlösliche Anteil aus Methanol/Wasser (20:7,5) an Kieselgel chromatographiert und das ölige Eluat erneut an Kieselgel mit dem Laufmittelgemisch Methanol/Wasser/Essigsäure (20:7,5:2,5) chromatographiert.

Fp.: 149°C, Ausbeute: 4,5 g (69% der Theorie).
$C_{16}H_{26}N_4O_4$ (338,42)
berechnet: C 56,78%, H 7,74%, N 16,55%;
gefunden: C 56,88%, H 7,68%, N 16,67%.

## Beispiel 6

4-Methyl-1,7-bis(2-oxo-1-pyrrolidinacetyl)-4-aza-1,7-diaminoheptan

8,55 g (0,05 Mol) Äthyl-2-oxo-1-pyrrolidinacetat und 3,63 g (0,025 Mol) Methyldi-(3-aminopropyl)amin werden unter Feuchtigkeitsausschluß in 5 ml absolutem Methanol 22 Stunden lang gekocht. Nach dem Abdampfen des Lösungsmittels wird der Rückstand aus dem Laufmittelgemisch Methanol/Wasser/Ammoniak (80:20:1) an Kieselgel chromatographiert. Das Eluat wird eingedampft, der Lösungsrückstand in Dichlormethan aufgenommen, mit Aktivkohle versetzt, geschüttelt, filtriert, das Filtrat abgedampft und der Rückstand getrocknet. Es handelt sich um ein öliges Produkt von $n_D^{54}$ 1,5135.

Ausbeute: 5,2 g (52,5% der Theorie)
$C_{19}H_{33}N_5O_4$ (448,57)
berechnet: C 52,54%, H 7,69%, N 15,61%;
gefunden: C 52,29%, H 8,10%, N 15,98%.

## Beispiel 7

2,2,4-Trimethyl-1,6-bis(2-oxo-1-pyrrolidinacetyl)-1,6-diaminohexan

8,55 g (0,05 Mol) Äthyl-2-oxo-1-pyrrolidinacetat und 3,95 g (0,025 Mol) 2,2,4-Trimethyl-1,6-diaminhexan werden in 5 ml absolutem Methanol unter Feuchtigkeitsausschluß 22 Stunden gekocht. Der Rückstand nach Abdampfen des Lösungsmittels wird aus Methanol/Wasser (80:30) an Kieselgel chromatographiert. Eine zweite Chromatographie erfolgt anschließend aus Methanol/Wasser/Essigsäure (20:7,5:2,5) und schließlich die Entfernung des Wassers und der Essigsäure durch Chromatographie an Aluminiumoxid. Es handelt sich um ein sirupöses Produkt mit $n_D^{54}$ 1,5095.
Ausbeute: 5,8 g (57% der Theorie)
$C_{21}H_{36}N_4O_4 \cdot 0,5\ H_2O$ (417,55)
berechnet: C 60,40%, H 8,93%, N 13,41%;
gefunden: C 60,35%, H 9,09%, N 13,41%.

Beispiel 8

1,4-Bis(2-oxo-1-pyrrolidinacetamido)cyclohexan

9,66 g (0,03 Mol) 2,4,5-Trichlorphenyl-2-oxo-1-pyrrolidinacetat werden in 150 ml Dimethylformamid gelöst und bei −5°C mit einer Lösung von 1,71 g (0,015 Mol) 1,4-Diaminocyclohexan in 20 ml Dimethylformamid unter Feuchtigkeitsausschluß versetzt. Nach viertelstündigem Stehen bei 0°C und weiteren drei Stunden bei Raumtemperatur wird das Lösungsmittel abgedampft, der Rückstand mit Aceton behandelt und der ausgefallene Niederschlag abfiltriert, mit Äther gewaschen und getrocknet.
Fp.: 294°C (Methanol-Äther), Ausbeute: 4,1 g (75% der Theorie)
$C_{18}H_{28}N_4O_4$ (364,45)
berechnet: C 59,32%, H 7,74%, N 15,37%;
gefunden: C 59,24%, H 7,81%, N 15,34%.

Beispiel 9

$\alpha,\alpha'$-Bis(2-oxo-1-pyrrolidinacetamido)-m-xylol

Analog Beispiel 8 durch Reaktion von 12,8 g (0,04 Mol) 2,4,5-Trichlorphenyl-2-oxo-1-pyrrolidinacetat und 2,72 g (0,02 Mol) $\alpha,\alpha'$-Diamino-m-xylol, und Reinigen aus Methanol an Kieselgel.
Fp.: 169—170°C, Ausbeute: 6,6 g (88% der Theorie).
$C_{20}H_{26}N_4O_4$ (386,46)
berechnet: C 62,15%, H 6,78%, N 14,49%;
gefunden: C 62,14%, H 6,84%, N 14,53%.

Beispiel 10

1,2-Bis(2-oxo-1-pyrrolidinacetyl)-1,2-propylendiamin

Analog Beispiel 9 aus 12,88 g (0,04 Mol) 2,4,5-Trichlorphenyl-2-oxo-1-pyrrolidinacetat und 1,48 g (0,02 Mol) 1,2-Diaminopropan.
Fp.: 147—148°C, Ausbeute: 4,3 g (66,5% der Theorie).
$C_{15}H_{24}N_4O_4$ (324,39)
berechnet: C 55,54%, H 7,45%, N 17,27%;
gefunden: C 55,58%, H 7,57%, N 17,31%.

Beispiel 11

2,6-Bis(2-oxo-1-pyrrolidinacetamido)pyridin

0,05 Mol 2-(2-Oxo-1-pyrrolidin)acetylchlorid werden in 200 ml Dichlormethan gelöst und bei −5°C mit 0,25 Mol 2,6-Diaminopyridin in Gegenwart von Triäthylamin unter Feuchtigkeitsausschluß umgesetzt. Nach einstündigem Stehen bei +10°C wird das Lösungsmittel im Vakuum abgedampft, der Rückstand mit Aceton behandelt und anschließend über Kieselgel (Methanol:Wasser; 100:25) chromatographisch gereinigt.
Fp.: 240—242°C, Ausbeute: 58% der Theorie

Beispiel 12

2,6-Bis(2-oxo-1-pyrrolidinacetamido)hexansäureäthylester

0,02 Mol L-2,6-Diamino-hexansäureäthylester werden in 10 ml Wasser gelöst und mit 0,02 Mol 4-Äthyl-morpholin als Säurebinder und mit 0,04 Mol 2,4,5-Trichlorphenyl-2-oxo-1-pyrrolidinacetat in 80 ml Dioxan bei 20°C zur Umsetzung gebracht. Die Reaktionslösung wird 20 Stunden bei 22° stehengelassen und anschließend das Lösungsmittel abgedampft. Der Rückstand wird in einem Gemisch von Äthanol und Dichloräthan (1:1) aufgenommen und über Kieselgel chromatographisch gereinigt.
Gelblicher Sirup, Ausbeute: 48% der Theorie
Brechungsindex $[\alpha]_D^{20}$ − 10,55° (c=2 cm).

Beispiel 13

2,6-Bis(2-oxo-1-pyrrolidinacetamidomethyl)pyridin

0,02 Mol 2,4,5-Trichlorphenyl-2-oxo-1-pyrrolidinacetat werden in 50 ml Dimethylformamid gelöst und bei −5°C mit einer Lösung von 0,01 Mol 2,6-Diaminomethyl-pyridin, gelöst in 20 ml Dimethylformamid, unter Rühren und unter Feuchtigkeitsausschluß umgesetzt. Nach dreistündigem Stehen bei Zimmertemperatur wird die Reaktionslösung 2 Stunden auf 60°C erwärmt, anschließend wird das Lösungsmittel abgedampft, der Rückstand mit Äther versetzt und die ausfallende Kristalle abfiltriert.
Fp.: 149°C, Ausbeute: 57% der Theorie

Ausgangsverbindung:
2,4,5-Trichlorphenyl-2-oxo-1-pyrrolidinacetat

84,8 g 2-Oxo-1-pyrrolidinessigsäure und 128,5 g 2,4,5-Trichlorphenol werden in 1500 ml Dichlormethan gelöst, langsam bei 0°C mit 138,75 g Dicyclohexylcarbodiimid versetzt und drei Stunden lang bei 20°C gerührt. Anschließend wird der ausgefallene Harnstoff abfiltriert, das Lösungsmittel abgedampft und der Rückstand aus Äthylacetat rekristallisiert.

Fp.: 128°C, Ausbeute: 188 g.

$C_{12}H_{10}Cl_3NO_3$ (322,58)

berechnet: C 44,68%, H 3,12%, Cl 32,9% N 4,34%;
gefunden: C 44.66%, H 3,14%, Cl 32,94% N 4,49%.

**Patentansprüche**

1. 2-Oxo-1-pyrrolidinessigsäurederivate der allgemeinen Formel

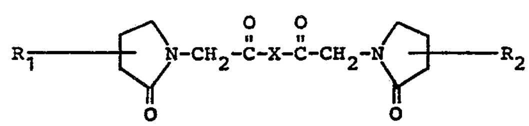

(I)

in welcher

$R_1$ und $R_2$ jeweils für Wasserstoff, Methyl, Methoxy, Fluor oder Chlor stehen,

X für die Gruppierung $-\underset{R_3}{\underset{|}{N}}-Y-\underset{R_4}{\underset{|}{N}}-$ steht,

wobei diese Gruppierung einschließlich der beiden Stickstoffatome entweder für einen Heterozyklus mit 5,6 oder 7 Ringgliedern steht,

oder wobei

$R_3$ und $R_4$ jeweils Wasserstoff oder Methyl bedeutet und

Y für eine Phenylen- oder eine Pyridylengruppe steht

oder für eine Alkylenarylenalkylengruppierung steht, in welcher die Alkylengruppen 1 oder 2 Kohlenstoffatome enthalten und die Arylengruppe eine Phenylen- oder Pyridylengruppe bedeutet

oder für eine Cycloalkylengruppe mit 5,6 oder 7 Ringgliedern steht

oder für eine geradkettige oder verzweigte Alkylengruppe mit 2 bis 9 Kohlenstoffatomen steht die gegebenenfalls durch eine Äthoxycarbonylgruppe substituiert ist und in welcher gegebenenfalls eine $CH_2$-Gruppe durch eine N-Alkyl (1—2 C-Atome) substituiert ist,

und ihre physiologisch verträglichen Salze mit Säuren.

2. Verfahren zur Herstellung von 2-Oxo-1-pyrrolidinessigsäurederivate gemäß Formel (I) in Anspruch 1, dadurch gekennzeichnet, daß man Verbindungen der allgemeinen Formel (II)

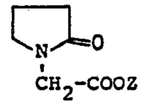

(II)

in welcher

Z eine niedere Alkylgruppe oder eine aktivierte Estergruppierung, bedeutet,

mit einer Verbindung der allgemeinen Formel

$$H—X—H \quad (III)$$

in welcher

X die oben angeführte Bedeutung hat,

gegebenenfalls in Gegenwart von inerten organischen Lösungsmitteln und gegebenenfalls in Gegenwart einer Base, umsetzt.

3. Arzneimittel enthaltend mindestens ein 2-Oxo-1-pyrrolidinessigsäurederivat gemäß Formel (I) in Anspruch 1.

4. Verfahren zur Herstellung von Arzneimitteln, dadurch gekennzeichnet, daß man Verbindungen gemäß Formel (I) in Anspruch 1, gegebenenfalls unter Zusatz von interten pharmazeutisch unbedenklichen Hilfs- und Trägerstoffen in eine geeignete Applikationsform überführt.

**Claims**

1. 2-Oxo-1-pyrrolidineacetic acid derivatives of the general formula (I)

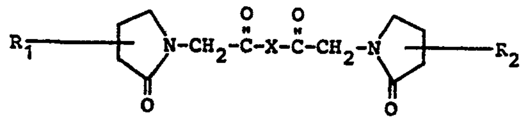

(I)

in which

$R_1$ and $R_2$ each denote hydrogen, methyl, methoxy, fluorine or chlorine

X represents the groups 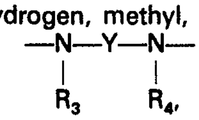

and this grouping, including the two nitrogen atoms, either represents a heterocyclic ring with 5, 6 or 7 ring members,

or

$R_3$ and $R_4$ each denote hydrogen or methyl,

and

Y denotes a phenylene or a pyridylene group

or it denotes an alkylenearylenealkylene grouping, in which the alkylene groups contain 1 or 2 carbon atoms and the arylene group denotes a phenylene or pyridylene group

or it represents a cycloalkylene group with 5, 6 or 7 ring members

or it represents a straight-chained or branched alkylene group with 2 to 9 carbon atoms which is optionally substituted by an ethoxy carbonyl group and in which optionally a $CH_2$-group is substituted by an N-alkyl (with 1 or 2 C-atoms),

and their physiologically compatible salts with acids.

2. A process for the preparation of 2-oxo-1-pyrrolidineacetic acid derivatives according to formula (I) in claim 1, characterized in that compounds of the general formula (II)

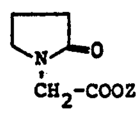

(II)

in which

Z denotes a lower alkyl group or

an activated ester grouping,

are reacted with a compound of the general formula

H—X—H (III)

in which

X has the above-mentioned meaning,

optionally in the presence of inert organic solvents and optionally in the presence of a base.

3. Medicaments containing at least one 2-oxo-1-pyrrolidineacetic acid derivative according to formula (I) in claim 1.

4. A process for the production of medicaments, characterized in that compounds of formula (I) in claim 1 are converted into a suitable administration form optionally by the addition of inert pharmaceutically acceptable auxiliaries and excipients.

**Revendications**

1. Dérivés d'acide 2-oxo-1-pyrrolidine-acétique de formule générale (I)

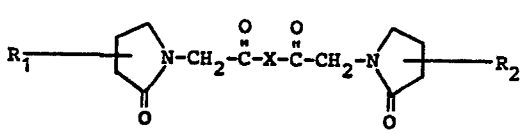

(I)

dans laquelle

$R_1$ et $R_2$ représentent chacun de l'hydrogène, méthyle, méthoxy, fluor ou chlore,
X représente le groupement —N—Y—N—

$$\begin{array}{ccc} -N- & Y & -N- \\ | & & | \\ R_3 & & R_4 \end{array}$$

ce groupement représentant, y compris les deux atomes d'azote, soit un hétérocycle ayant 5, 6 ou 7 chaînons nucléaires,
ou
$R_3$ et $R_4$ représentant chacun de l'hydrogène ou un méthyle, tandis que
Y représente un groupe phénylène ou pyridylène, ou représente un groupement alcoylène-arylène-alcoylène dans lequel les groupes alcoylène contiennent 1 ou 2 atomes de carbone et le groupe arylène représente un groupe phénylène ou pyridylène,
ou un groupe cycloalcoylène ayant 5, 6 ou 7 chaînons nucléaires,
ou représente un groupe alcoylène à chaîne droite ou ramifée ayant 2 à 9 atomes de carbone, qui est éventuellement substitué par un groupe éthoxycarbonyle et dans lequel éventuellement un groupe $CH_2$ est substitué par un N-alcoyle (à 1—2 atomes de carbone),
et leurs sels, physiologiquement compatibles, avec des acides.

2. Procédé de préparation des dérivés d'acide 2-oxo-1-pyrrolidine-acétique de formule (I) selon la revendication 1, caractérisé en ce qu'on fait réagir des composés de formule générale (II)

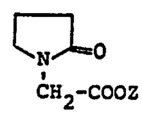

(II)

dans laquelle
Z représente un groupe alcoyle inférieur ou un groupement ester activé,
avec un composé de formule générale:

$$H—X—H \qquad (III)$$

dans laquelle
X a la signification indiquée plus haut,
éventuellement en présence de solvants organiques inertes et éventuellement en présence d'une base.

3. Médicaments contenant au moins un dérivé d'acide 2-oxo-1-pyrrolidine-acétique selon la formule (I) de la revendication 1.

4. Procédé de préparation de médicaments, caractérisé en ce qu'on convertit des composés selon la formule (I) de la revendication 1, éventuellement avec addition de substances auxiliaires et de support inertes, pharmaceutiquement inoffensives, en une forme d'application appropriée.